# EUROPEAN PATENT APPLICATION

(11) **EP 1 834 644 A2**
(43) Date of publication of application: **19.09.2007**
(21) Application number: 07090052.7
(22) Date of filing: 27.05.2004
(51) Int. Cl.: A61K 31/567, A61P 5/32, A61P 5/36

(54) **Composition comprising antiprogestins and pure antiestrogens for prophylaxis and treatment of hormone-dependent diseases**

(30) Priority: 28.05.2003 US 446165
(62) Divisional of application: 04739401.0
(71) Applicant: Bayer Schering Pharma Aktiengesellschaft, 13353 Berlin (DE)
(72) Inventor: Fuhrmann, Ulrike, 10587 Berlin (DE); Hoffmann, Jens, 16567 Mühlenbeck (DE); Schneider, Martin, 13469 Berlin (DE); Siemeister, Gerhard, 13503 Berlin (DE)

(57) **Abstract**

The present invention relates to methods and uses for preventing or treating hormone-dependent diseases, in particular breast cancer, in a mammal, by a combination of an progesterone-receptor antagonist, in particular the progesterone-receptor antagonist 11β-(4-acetylphenyl)-17β-hydroxy-17α-(1,1,2,2,2-pentafluoroethyl)-estra-4,9-dien-3-one or a pharmaceutically acceptable derivative or analogue thereof, and a pure antiestrogen, in particular a compound of general formula I as defined in the specification, for instance 11β-Fluoro-17α-methyl-7α-{5-[methyl(8,8,9,9,9-pentafluorononyl)amino]pentyl}-estra-1,3,5(10)-triene-3,17β-diol. The invention further relates to pharmaceutical compositions comprising said combination.

## Description

### Field of the Invention

The present invention relates to the use of a combination comprising an progesterone-receptor antagonist, in particular 11β-(4-acetylphenyl)-17β-hydroxy-17α-(1,1,2,2,2-pentafluoroethyl)-estra-4,9-dien-3-one or a pharmaceutically acceptable derivative or analogue thereof, and a pure antiestrogen for the prophylaxis and treatment of hormone-dependent diseases (for example, estrogen- and progesterone-dependent diseases), such as breast cancer. The present invention further relates to pharmaceutical compositions comprising a combination of an progesterone-receptor antagonist and a pure antiestrogen for the prophylaxis and treatment of hormone-dependent diseases, such as breast cancer.

### Background of the Invention

Endocrine therapy represents a mainstay of effective, minimally toxic, palliative treatment for metastatic breast cancer. As a standard palliative treatment of non-operable mammary carcinomas as well as for adjuvant therapy after primary treatment of mammary carcinomas, antiestrogens, such as the non-steroidal antiestrogen tamoxifen, are used. However, tamoxifen cannot cure breast cancer. Thus, for secondary therapy progestins or aromatase inhibitors are commonly used. In premenopausal women ovariectomy, tamoxifen and LHRH (luteinizing hormone releasing hormones) analogs achieve comparable results (H.T. Mouridson et al., Eur. J. Cancer Clin. Oncol., 24, pp. 99-105, 1988). Although tamoxifen is widely used for adjuvant therapy of breast cancer, its use as a chemopreventive agent is problematic, because it has been shown that the treatment results in an increase in the incidence of endometrial cancers (I.N. White, Carcinogenesis, 20(7):1153-60, 1999; L. Bergman et al., The Lancet, Vol. 356, Sept. 9, 2000).

WO 98/07740 discloses 7α-(ξ-aminoalkyl)-estratrienes having strong antiestrogenic activity. Some of them are pure antiestrogens, others are partial antiestrogens (like e.g. the non-steroidal antiestrogens tamoxifen and raloxifen), i.e. they exhibit a partial estrogenic effect. The 7α-(ξ-aminoalkyl)-estratrienes according to WO 98/07740 are reported to be suitable for tumor therapy and hormone substitution therapy.

WO 99/33855 discloses 11β-halogen-7α-substituted estratrienes having either pure or partial antiestrogenic activity. The additional 11β-halogen atom is made responsible for the properties of the antiestrogens according to WO 99/33855, which are also reported to be suitable for the treatment of hormone-dependent tumors and conditions.

The non-prepublished DE 101 59 217.5 (filing date Nov. 27, 2001) and the corresponding PCT/EP02/13484 and U.S. Patent Application No. 10/305,418, which are all enclosed herewith by reference, describe 17α-alkyl-17β-oxy-estratrienes, their production, their use for the production of pharmaceutical agents as well as pharmaceutical preparations containing them.
These compounds have the general formula 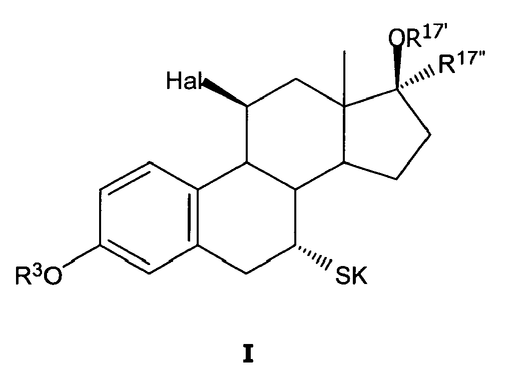 in which
- Hal: stands for F or Cl, and is bonded to the estratriene skeleton in 11β-position,

- R³: stands for hydrogen, C₁- - C₄-alkyl, C₁- - C₄-alkanoyl or a cyclic C₃- - C₇-ether with an O atom,
- R^{17'}: stands for hydrogen, C₁- - C₄-alkyl or C₁- - C₄-alkanoyl,
- R^{17"}: stands for C₁- - C₄-alkyl, C₁- - C₄-alkyl, C₁- - C₄-alkinyl as well as for at least partially fluorinated C₁- - C₄-alkyl radicals,
whereby R^{17'}-O in 17β-position and R^{17"} in 17α-position are bonded to the estratriene skeleton, and
- SK: stands for the grouping U-V-W-X-Y-Z-E, whereby this grouping is bonded to the estratriene skeleton via U in 7α-position,
in which U represents either a straight-chain or branched-chain C₁- - C₁₃-alkylene-, -alkenylene- or -alkinylene radical or the group A-B, whereby A is bonded to the estratriene skeleton and represents a benzylidene radical that is bonded via -CH₂- to the estratriene skeleton, a phenylene radical, or a C₁- - C₃-alkylaryl radical that is bonded via the alkyl group to the estratriene skeleton, and B stands for a straight-chain or branched-chain C₁ - - C₁₃-alkylene-, -alkenylene- or -alkinylene radical, and whereby A and B can also be connected to one another via an O atom,
in which V further represents a CH₂- or a C(O) group,
in which W further is an N(R⁶)- group or an N⁺(O⁻)(R⁶) group or an azolidinylene ring or an azolidinylene-N-oxide ring, whereby the azolidinylene ring or azolidinylene-N-oxide ring includes at least one C atom of grouping X, whereby
R⁶ further is either H or CH₂-R⁷ or C(O)-R⁷, in which R⁷ can mean the following:
a) hydrogen or
b) a straight-chain or branched-chain, non-fluorinated or at least partially fluorinated C₁- - C₁₄-alkyl-, -alkenyl- or-alkinyl radical, which can be hydroxylated in one or more places and can be interrupted by one to three of the heteroatoms -O- and -S- and/or the groupings -NR⁹-, in which R⁹ stands for hydrogen or a C₁- - C₃-alkyl radical, or
c) an unsubstituted or substituted aryl- or heteroaryl radical or
d) an unsubstituted or substituted C₃- - C₁₀-cycloalkyl radical or
e) an unsubstituted or substituted C₄- - C₁₅-cycloalkylalkyl radical or
f) an unsubstituted or substituted C₇- - C₂₀-aralkyl radical or
g) an unsubstituted or substituted heteroaryl-C₁- - -C₆-alkyl radical or
h) an unsubstituted or substituted aminoalkyl radical or a biphenyl radical,
in which X further is a straight-chain or branched-chain C₁- - C₁₂-alkylene-,-alkenylene- or -alkinylene radical,
in which Y further is a direct bond between X and Z or can mean the following:
a) an SOₙ-R¹⁰ group, whereby n = 0, 1 or 2, only if W is an N⁺(O⁻)(R⁶) group or an azolidinylene-N-oxide ring and not an N(R⁶) group or an azolidinylene ring,
   whereby R¹⁰ represents a direct bond between SOₙ and Z or a straight-chain or branched-chain C₁- - C₆-alkylene-, -alkenylene- or -alkinylene radical, or
b) the group R¹¹ or O-R¹¹, whereby R¹¹ stands for
   i) a straight-chain or branched-chain C₁- - C₅-alkylene-, -alkenylene- or -alkinylene radical or for
   ii) an unsubstituted or substituted aryl radidal or heteroaryl radical or for
   iii) an unsubstituted or substituted C₃- - C₁₀-cycloalkyl radical or for
   iv) an unsubstituted or substituted C₄- - C₁₅-cycloalkylalkyl radical or for
   v) an unsubstituted or substituted C₇- - C₂₀-aralkyl radical or for
   vi) an unsubstituted or substituted heteroaryl-C₁- - -C₆-alkyl radical, or
c) the grouping CH = CF or
d) the grouping HN-C(O)-NH-R¹², whereby R¹² stands for an unsubstituted or substituted arylene radical, and whereby R¹² is bonded to Z, and in which Z further is a direct bond between Y and E or a straight-chain or branched-chain C₁- - C₉-alkylene-, -alkenylene- or -alkinylene radical, which can be partially or completely fluorinated, and
   in which E further is a CF₃ group or an at least partially fluorinated aryl group,
whereby pharmacologically compatible acid addition salts as well as esters are also included.

Hal in particular stands for fluorine.

R³ can be hydrogen, methyl, ethyl, *n*-propyl, *iso*-propyl, *n*-butyl, *iso*-butyl and *tert*-butyl, a corresponding alkanoyl (acetyl, propionyl, butanoyl) or a cyclic ether. R³ in particular stands for hydrogen, CH₃, CH₃CO or C₅H₁₀O.
R^{17'} and R^{17"} are in particular methyl, ethyl, *n*-propyl, *iso*-propyl, *n*-butyl, *iso*-butyl and *tert*-butyl, whereby R^{17'} in addition can also be hydrogen, acetyl, propionyl and butanoyl, and whereby in this case, the corresponding isomers can be included. In addition, R^{17"} can be ethinyl, 1-propinyl, 2-propinyl, 1-butinyl, 2-butinyl and 3-butinyl as well as trifluoromethyl, pentafluoroethyl, heptafluoropropyl and nonafluorobutyl, whereby in this case, the corresponding isomers are also included. R^{17'} is in particular hydrogen, CH₃ or CH₃CO. R^{17"} preferably stands for methyl, ethinyl and trifluoromethyl.
U can be in particular a straight-chain or branched-chain alkylene radical and in particular a methylene, ethylene, propylene, butylene, pentylene, hexylene, heptylene, octylene, nonylene, decylene, undecylene, dodecylene or tridecylene radical. U preferably stands for (CH₂)ₚ, whereby p is an integer from 2 to 10. In particular, U is preferably a butylene, pentylene, hexylene or heptylene radical. U is quite especially preferably an *n*-butylene radical, i.e., in the formula (CH₂)ₚ for U, p = 4.
In particular, V stands for CH₂. The grouping U-V thus can be *n*-pentylene in a quite preferred embodiment.
In particular, W stands for the amine-N-oxide N⁺(O⁻)(R⁶) or for the amine N(R⁶), whereby R⁶ is preferably hydrogen or CH₂-R⁷, in which R⁷ stands in particular for hydrogen or methyl or ethyl. R⁶ is thus preferably hydrogen or a C₁- - C₃-alkyl radical, thus in particular a methyl, ethyl, *n*-propyl or *iso*-propyl radical. In an especially preferred embodiment, W represents an N⁺(O⁻)(CH₃) group (N-methylamine-N-oxide).
X preferably stands for (CH₂)_{q}, whereby q = 0 or an integer from 1 to 12, thus for a direct bond between W and Y or for a straight-chain or branched methylene, ethylene, propylene, butylene, pentylene, hexylene, heptylene, octylene, nonylene, decylene, undecylene or dodecylene radical. In an especially preferred embodiment, X is an ethylene, *n*-propylene, *n*-butylene, *n*-pentylene, *n*-hexylene, *n-*heptylene or *n*-octylene radical.
In particular, Y can represent a direct bond between X and Z. If this is the case, X stands for a longer alkylene chain, thus in particular, X stands for *n*-hexylene, *n*-heptylene or *n-*octylene. In a preferred embodiment, Y can also be an SOₙ group, whereby n = 0, 1 or 2, thus a sulfanyl group, a sulfinyl group or a sulfonyl group. If Y is an SOₙ group, X represents a rather shorter alkylene chain, in particular an *n*-propyl chain.
Z is preferably a direct bond between Y and E or a straight-chain or branched-chain C₁- - C₇-alkylene radical, which can be at least partially fluorinated. In particular, Z can be a methylene, ethylene, propylene or butylene radical, which can be at least partially fluorinated. In particular, Z is difluoromethylene or a straight-chain alkylene radical, which is perfluorinated on one end, thus, for example, a 1,1-difluoroethylene, 1,1,2,2-tetrafluoro-n-propylene or 1,1,2,2,3,3-hexafluoro-n-butylene radical. Alkylene radicals that carry only two fluorine atoms on a terminal C-atom are especially advantageous, whereby this CF₂ group is bonded to radical E. In this case, side chain SK is terminated with C₂F₅.
In particular, E stands for CF₃ or for pentafluorophenyl. The grouping Z-E thus preferably represents one of the groups that is selected from the group that comprises C₂F₅, C₃F₇ and C₄F₉ as well as C₆F₅.
According to this invention, pharmacologically compatible acid addition salts as well as esters of 17α-alkyl-17β-oxy-estratrienes are also included. The addition salts are the corresponding salts with inorganic and organic acids. As addition salts, in particular the hydrochlorides, hydrobromides, acetates, citrates, oxalates, tartrates and methanesulfonates are considered. If R³ and R^{17'} are hydrogen, such that a 3,17β-diol is present, the esters of these hydroxy compounds can also be formed. These esters are preferably formed with organic acids, whereby the same acids as for forming the addition salts are suitable, namely in particular acetic acid, but also higher carboxylic acids, such as, e.g., propionic, butyric, isobutyric, valeric, isovaleric or pivalic acid.

As special compounds the following are mentioned
11β-Fluoro-7α-{5-[methyl(7,7,8,8,9,9,9-heptafluorononyl)amino]pentyl}-17α-methylestra-1,3,5(10)-triene-3,17β-diol N-oxide
11β-Fluoro-7α-{5-[methyl(8,8,9,9,10,10,10-heptafluorodecyl)amino]pentyl}-17α-methylestra-1,3,5(10)-triene-3,17β-diol N-oxide
(RS)-11β-Fluoro-7α-{5-[methyl(7,7,8,8,9,9,10,10,10-nonafluorodecyl)amino]-pentyl}-17α-methylestra-1,3,5(10)-triene-3,17β-diol N-oxide
11β-Fluoro-7α-{5-[methyl(8,8,9,9,9-pentafluorononyl)amino]pentyl}-17α-methylestra-1,3,5(10)-triene-3,17β-diol N-oxide
11 β-Fluoro-7α-{5-[methyl(9,9,10,10,10-pentafluorodecyl)amino]pentyl-17α-methylestra-1,3,5(10)-triene-3,17β-diol N-oxide
11 β-Fluoro-7α-{5-[methyl(8,8,9,9,9-pentafluorononyl)amino]pentyl}-17α-methylestra-1,3,5(10)-triene-3,17β-diol
11β-Fluoro-7α-{5-[methyl(7,7,8,8,9,9,10,10,10-nonafluorodecyl)amino]pentyl}-17α-methylestra-1,3,5(10)-triene-3,17β-diol
11β-Fluoro-7α-{5-[methyl(7,7,8,8,9,9,9-heptafluorononyl)amino]pentyl}-17α-methylestra-1,3,5(10)-triene-3,17β-diol
17α-Ethinyl-11β-fluoro-7α- {5-[methyl(7,7,8,8,9,9,10,10,10-nonafluorodecyl)-amino]pentyl}-estra-1,3,5(10)-triene-3,17β-diol
17α-Ethinyl-11β-fluoro-3-(2-tetrahydropyranoyloxy)-7α-{5-[methyl(7,7,8,8,9,9,10,10,10-nonafluorodecyl)amino]pentyl}-estra-1,3,5(10)-trien-17β-ol
11β-Fluoro-3-(2-tetrahydropyranyloxy)-7α-{5-[methyl(7,7,8,8,9,9,10,10,10-nonafluorodecyl)amino]pentyl}-17α-methylestra-1,3,5(10)-trien-17β-ol
11β-Fluoro-7α-{5-[methyl(7,7,8,8,9,9,10,10,10-nonafluorodecyl)amino]pentyl}-17α-trifluoromethylestra-1,3,5(10)-triene-3,17β-diol
11β-Fluoro-7α-{5-[methyl(6,6,7,7,8,8,8-heptafluorooctyl)amino]pentyl}-17α-methylestra-1,3,5(10)-triene-3,17β-diol
11β-Fluoro-7α-{5-[methyl(8,8,9,9,10,10,10-heptafluorodecyl)amino]pentyl}-17α-methylestra-1,3,5(10)-triene-3,17β-diol
11β-Fluoro-7α-{5-[methyl(6,6,7,7,8,8,9,9,10,10,10-undecafluorodecyl)amino]-pentyl}-17α-methylestra-1,3,5(10)-triene-3,17β-diol
11β-Fluoro-7α-{5-[methyl(5,5,6,6,7,7,8,8,8-nonafluorooctyl)amino]pentyl}-17α-methylestra-1,3,5(10)-triene-3,17β-diol
11β-Fluoro-7α-{5-[methyl(9,9,10,10,11,11,11-heptafluoroundecyl)amino]-pentyl}-17α-methylestra-1,3,5(10)-triene-3,17β-diol
11β-Fluoro-7α-{5-[methyl(9,9,10,10,10-pentafluorodecyl)amino]pentyl}-17α-methylestra-1,3,5(10)-triene-3,17β-diol.

These compounds show high antiestrogenic activity after oral administration. Due to the blockade of the 17α-position formation of (active) metabolites is suppressed.

Progesterone-receptor antagonists (also termed as antiprogestins) represent a relatively new and promising class of therapeutic agents that could have significant impact on breast cancer treatment. Certain progesterone-receptor antagonists have recently gained importance in the endocrine therapy of those breast cancers possessing receptors for progesterone (T. Maudelonde et al., in: J.G.M. Klijn et al., Hormonal Manipulation of Cancer: Peptides, Growth Factors and New (Anti) Steroidal Agents, Raven Press, New York, 1987, pp. 55-59). This new strategy in endocrine therapy is based on the antitumor activity of progesterone-receptor antagonists in progesterone receptor-positive human breast cancer cell lines *in vitro* and in several hormone-dependent mammary tumors of the mouse and rat *in vivo.* In particular, the antitumor mechanism of the progesterone-receptor antagonists onapristone and mifepristone (RU 486) was investigated using the hormone-dependent MXT mammary tumor model of the mouse as well as the DMBA- and the MNU-induced mammary tumor models of the rat (M. R. Schneider et al., Eur. J. Cancer Clin. Oncol., Vol. 25, No. 4, pp. 691-701, 1989; H. Michna et al., Breast Cancer Research and Treatment 14:275-288, 1989; H. Michna, J. Steroid. Biochem. Vol. 34, Nos 1-6, pp. 447-453, 1989). However, due to low activity and adverse side effects involved with e.g. mifepristone these compounds could not be recommended as a single agent in the management of breast cancer (D. Perrault et al., J. Clin. Oncol. 1996 Oct, 14(10), pp.2709-2712).

Another problem with for instance RU 486 was poor bioavailability when administered orally. Thus, they generally had to be administered in high doses, giving rise to possible unfavorable side effects. Moreover, oral administration is desirable with respect to patient convenience and compliance.

Furthermore, there is still a need for compounds that are active not only in the treatment, but also in the prophylaxis of breast cancer and other hormone-dependent diseases.

In the prior art, progesterone-receptor antagonists have furthermore been utilized for initiating abortions and thus for use in postcoital fertility control, as contraceptives for women (WO-A 93/23020, WO-A 93/21927) and further for the treatment of hormone irregularities, for initiating menstruation and for initiating labor. Further indications are in the field of hormone substitution therapy (WO-A 94/18983), the treatment of afflictions related to dysmenorrhea and the treatment of endometriosis (EP-A 0 266 303) as well as myomas.

17α-fluoroalkylsteroids having progesterone-receptor antagonist activity as well as methods for producing them are described in WO 98/34947.

It has been found that the growth of hormone-dependent tumors depend, among others, e.g. on estrogens, progesterones and even testosterones. For example, most mammary carcinomas exhibit estrogen as well as progesterone receptors. Thus, a combination of progesterone-receptor antagonists and antiestrogens may be effective in the therapy of pre- and postmenopausal mammary carcinomas.

EP 0 310 542 B1 teaches the use, in very general terms, of a combination of progesterone-receptor antagonists and antiestrogens in a weight ratio of 1:50 to 50:1 for the preparation of a medicament for the treatment of hormone-dependent tumors. There is no specific disclosure to use pure antiestrogens and the progesterone-receptor antagonist 11β-(4-acetylphenyl)-17β-hydroxy-17α-(1,1,2,2,2-pentafluoroethyl)-estra-4,9-dien-3-one or a pharmaceutically acceptable derivative or analogue thereof in such combination.

Despite a general disclosure of the potential desirability of combining antiestrogens and progesterone-receptor antagonists, the prior art has failed to disclose a specific combination having superior advantages for the treatment of breast-cancer and other hormone-dependent conditions. Disadvantages of the prior art combinations mainly result from a weak activity of the antiestrogen part of the combination and from the partial estrogen agonism of certain antiestrogens (like, e.g., tamoxifen).

### Object of the Present Invention

It is the object of the present invention to prevent or reduce the disadvantages of the prior art, i.e. to provide a highly efficient prophylaxis and treatment of breast cancer and other diseases dependent upon hormones.

It is a further object of the present invention to provide pharmaceutical compositions comprising highly effective antitumor agents for the prophylaxis and treatment of breast cancer and other hormone-dependent diseases.

These objects are achieved by a new and inventive antiestrogen-progesterone-receptor antagonist combination comprising the progesterone-receptor antagonist 11β-(4-acetylphenyl)-17β-hydroxy-17α-(1,1,2,2,2-pentafluoroethyl)-estra-4,9-dien-3-one or a pharmaceutically acceptable derivative or analogue thereof and at least one pure antiestrogen. Surprisingly, this new and inventive combination is highly effective in inhibiting tumor growth. Moreover, the inhibition achieved is superior and even synergistic when compared to the inhibition of the progesterone-receptor antagonist and pure antiestrogens alone. In addition, it has been discovered that the combination according to the invention is accompanied by increased apoptosis of tumor cells, a particularly advantageous mechanism of action for the treatment of mammary carcinoma and other hormone-dependent diseases, where an indicator of high risk is an increased amount of tumor cells in the S-phase of the cell cycle. Such other hormone-dependent diseases may include ovarian cancer, endometrial cancer, myeloma, lung cancer, anovulatory infertility, meningoma, i.e., diseases which substantially originate or are influenced by the presence of hormone receptors and/or hormone-dependent pathways.

### Summary of the Invention

The present invention provides a pharmaceutical composition comprising the progesterone-receptor antagonist 11β-(4-acetylphenyl)-17β-hydroxy-17α-(1,1,2,2,2-pentafluoroethyl)-estra-4,9-dien-3-one or a pharmaceutically acceptable derivative or analogue thereof and at least one pure antiestrogen. Preferred pure antiestrogens are the antiestrogens of the general formula I 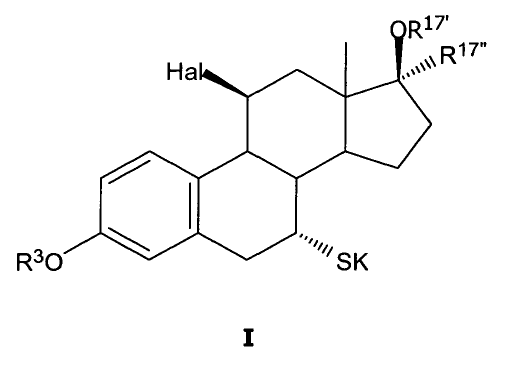 wherein the substituents have the meanings mentioned above.

The invention furthermore relates to the use of the above-mentioned combination for the preparation of a medicament for prophylaxis and treatment of breast cancer, as well as for the treatment of other hormone-dependent conditions. In particular, progesterone-receptor antagonist (I) is particularly suitable for preventing hormone-dependent tumors, and the combination of progesterone-receptor antagonist (I) with a pure antiestrogen has been shown to effectively inhibit the growth of such tumors as compared to the progesterone-receptor antagonist or pure antiestrogen alone.

In another aspect, the present invention provides a method for prophylaxis and treatment of breast cancer and other hormone-dependent diseases in a mammal, in particular a human, in need of such treatment, said method comprising administering a pharmaceutically effective amount of a composition comprising the progesterone-receptor antagonist 11β-(4-acetylphenyl)-17β-hydroxy-17α-(1,1,2,2,2-pentafluoroethyl)-estra-4,9-dien-3-one or a pharmaceutically acceptable derivative or analogue thereof, and at least one pure antiestrogen to a mammal in need thereof.

The preferred progesterone-receptor antagonist 11β-(4-acetylphenyl)-17β-hydroxy-17α-(1,1,2,2,2-pentafluoroethyl)-estra-4,9-dien-3-one will hereinafter be referred to as "progesterone-receptor antagonist (I)". The preferred pure antiestrogens of general formula I will hereinafter be referred to as "pure antiestrogens (I)".

### Detailed description of the Invention

### Progesterone-receptor antagonist (I) - 11β-(4-acetylphenyl)-17β-hydroxy-17α-(1,1,2,2,2-pentafluoroethyl)-estra-4,9-dien-3-one - is represented below by formula (I):

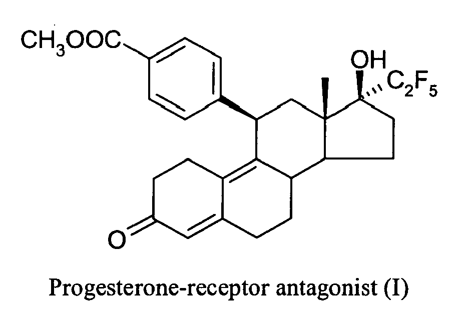

Progesterone-receptor antagonist (I) (or a pharmaceutically acceptable derivative or analogue thereof) is a valuable pharmaceutical agent which has strong progesterone-receptor antagonist activity. Progesterone-receptor antagonist (I) or a pharmaceutically acceptable derivative or analogue thereof can be used according to the present invention in combination with at least one pure antiestrogen. Preferably, the pure antiestrogen is selected from the group consisting of pure antiestrogens (I), including pharmaceutically acceptable derivatives or analogues of these pure antiestrogens. The combination is particularly advantageous for the prophylaxis and treatment of breast cancer and other hormone-dependent diseases.

Pure antiestrogens (I) are represented by the formula (I) below:

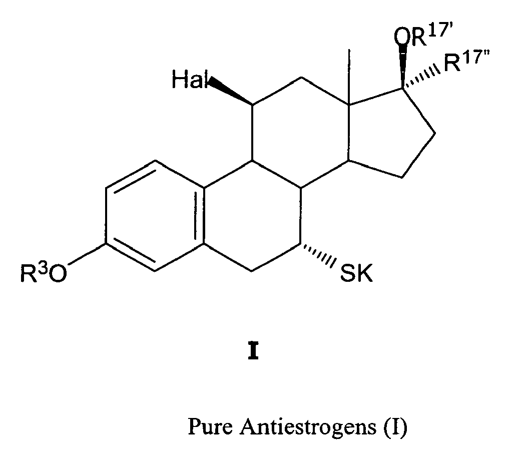

Amongst these, the compounds listed on pages 6, 7 and 8 are preferred; antiestrogen (Ia), 11β-Fluoro-17α-methyl-7α-{5-[methyl(8,8,9,9,9-pentafluorononyl)amino]pentyl}-estra-1,3,5(10)-triene-3,17β-diol is especially preferred.

A pharmaceutically acceptable derivative or analogue of progesterone-receptor antagonist (I) in the context of the present invention may include, for example, any one of the inventive compounds disclosed in WO 98/34947. A pharmaceutically acceptable derivative or analogue of pure antiestrogens (I) in the context of the present invention may include, for example, any one of the inventive compounds described in PCT/EP02/13484 (corresponding to U.S. Patent Application No. 10/305,418).
Also other compounds which are known as antiestrogens can be used for the purposes of the present invention, for instance faslodex (ICI 182,780).

The term "hormone-dependent diseases" in the context of the present invention includes, but is not limited to, e.g. breast cancer, ovarian cancer, endometrial cancer, endometriosis, myeloma, gastric cancer, anovulatory infertility, meningoma, i.e. diseases which substantially originate or are influenced by the presence of hormone receptors and/or hormone-dependent pathways.

Although progesterone-receptor antagonist (I) is the preferred progesterone-receptor antagonist for purposes of the present invention, this does not exclude the possibility to use other suitable progesterone-receptor antagonists as well.

As regards the superiority of the present invention over the prior art, it is especially favorable that e.g. in progesterone-receptor antagonist (I) used according to the present invention further endocrine side effects, such as e.g. androgen, estrogen or antiglucocorticoid activity are only very weak, if present at all.

Furthermore, the pure antiestrogens used according to the present invention have substantially no partial estrogen activity, compared to tamoxifen or raloxifen. The pure antiestrogens used according to the present invention, in particular pure antiestrogen (Ia) exhibit a particularly high bioavailability if compared to e.g. the conventionally used antiestrogen ICI 182,780 (EP-A-0 138 504). Due to the high bioavailability of the combination preparations according to the present invention comprising progesterone-receptor antagonist (I) and a pure antiestrogen (I), preferably pure antiestrogen (Ia) (including pharmaceutically acceptable derivatives or analogues of these pure antiestrogens), the medicament can be administered orally. Oral administration has the advantage of improved convenience and patient compliance. As a further favorable consequence, the progesterone-receptor antagonist-pure antiestrogen composition of the present invention is well tolerated. Partial agonism is commonly associated with undesirable side effects, such as for example in the case of the partial antiestrogen tamoxifen an increase in the incidence of endometrial cancers (see I.N. White, Carcinogenesis, 20(7):1153-60, 1999; L. Bergman et al., The Lancet, Vol. 356, Sept. 9, 2000, 881-887) as well as the antiglucocorticoid effects and certain toxic side effects related to the administration of the prior art progesterone-receptor antagonist mifepristone (see D. Perrault et al., J. Clin. Oncol. 1996 Oct, 14(10), pp.2709-2712; L.M. Kettel et al., Fertil. Steril. 1991 Sep, 56(3), pp.402-407; X. Bertagna, Psychoneuroendocrinology 1997, 22 Suppl. 1; pp. 51-55). Pure antiestrogens if used in the amounts according to the present invention will not show the undesired side effects associated with the partial antiestrogens.

In a first aspect, the present invention relates to a pharmaceutical composition comprising the progesterone-receptor antagonist (I) or a pharmaceutically acceptable derivative or analogue thereof, and at least one pure antiestrogen (I). The pure antiestrogen is preferably pure antiestrogen (Ia). Pharmaceutically acceptable derivatives or analogues of these preferred pure antiestrogens are also encompassed within the invention. Optionally, the progesterone-receptor antagonist and the pure antiestrogen can be further combined with other pharmacologically active agents. For example, they may also be combined with a cytotoxic agent.

In a second aspect, the present invention relates to the use of a composition comprising progesterone-receptor antagonist (I) or a pharmaceutically acceptable derivative or analogue thereof, and at least one pure antiestrogen (I) for the manufacture of a medicament, in particular for the prophylaxis or treatment of breast cancer or other hormone-dependent diseases. The pure antiestrogen is preferably pure antiestrogen (Ia). Pharmaceutically acceptable derivatives or analogues of these preferred pure antiestrogens are also encompassed within the invention.

In another aspect, the present invention relates to a method for the prophylaxis or treatment of breast cancer and other hormone-dependent diseases, comprising administering a composition comprising the progesterone-receptor antagonist (I) or a pharmaceutically acceptable derivative or analogue thereof and at least one pure antiestrogen (I) to a mammal, preferably a human, in need of such prophylaxis or treatment. The pure antiestrogen is preferably pure antiestrogen (Ia). Pharmaceutically acceptable derivatives or analogues of these preferred pure antiestrogens are also encompassed within this aspect of the invention.

Optionally, the pharmaceutical compositions, uses and methods according to the present invention further comprise other pharmacologically active agents. The manufacture of the medicaments/pharmaceutical compositions may be performed according to methods known in the art. Commonly known and used adjuvants as well as further suitable carriers or diluents may be used.
Suitable carriers and adjuvants may be such as recommended for pharmacy, cosmetics and related fields in: Ullmann's Encyclopedia of Technical Chemistry, Vol. 4, (1953), pp. 1-39*;* Journal of Pharmaceutical Sciences, Vol. 52 (1963), p. 918ff; H.v.Czetsch-Lindenwald, "Hilfsstoffe für Pharmazie und angrenzende Gebiete"; Pharm. Ind. 2, 1961, p.72ff; Dr. H.P. Fiedler, Lexikon der Hilfsstoffe für Pharmazie, Kosmetik und angrenzende Gebiete, Cantor KG, Aulendorf in Württemberg, 1971.

The combination of progesterone-receptor antagonist (I) and at least one pure antiestrogen (I), preferably pure antiestrogen (Ia) suitable for the purposes of the present invention can be incorporated into pharmaceutical compositions according to known methods of preparing galenics for oral, parenteral, e.g. intraperitoneal, intramuscular, subcutaneous or percutaneous application. They can also be implanted into tissue.

They can be administered in the form of tablets, pills, dragees, gel capsules, granules, suppositories, implants, injectable sterile aqueous or oily solutions, suspensions or emulsions, ointments, creams, gels, patches for transdermal administration, formulations suitable for administration by inhalation, for instance nasal sprays or by intravaginal (e.g. vaginal rings) or intrauterine systems (diaphragms, loops).

For the preparation of pharmaceutical compositions for oral administration, the active agents suitable for the purposes of the present invention as defined above can be admixed with commonly known and used adjuvants and carriers such as for example, gum arabic, talcum, starch, sugars like e.g. mannitose, methyl cellulose, lactose, gelatin, surface-active agents, magnesium stearate, aqueous or non-aqueous excipients, paraffin derivatives, crosslinking agents, dispersants, emulsifiers, lubricants, conserving agents and flavoring agents (e.g., ethereal oils). In the pharmaceutical composition, the progesterone-receptor antagonist and the pure antiestrogen may be dispersed in a microparticle, e.g. a nanoparticulate, composition.

In order to further enhance the bioavailability of the active agents, the active agents suitable for the purposes of the present invention as defined above can also be formulated as cyclodextrin clathrates by reacting them with α-, β- or γ-cyclodextrines or derivatives thereof according to the method as disclosed in PCT/EP95/02656. For parenteral administration the active agents suitable for the purposes of the present invention as defined above can be dissolved or suspended in a physiologically acceptable diluent, such as e.g., oils with or without solubilizers, surface-active agents, dispersants or emulsifiers. As oils for example and without limitation, olive oil, peanut oil, cottonseed oil, soybean oil, castor oil and sesame oil may be used.

The pharmaceutical compositions/medicaments according to the present invention can also be administered via a depot injection or an implant preparation, optionally for sustained delivery of the active agent(s).

Implants can comprise as inert materials e.g. biologically degradable polymers or synthetic silicones such as e.g. silicone rubber.

For percutaneous applications, the active agent(s) may also be formulated into adhesives.

The preferred mode of administration is oral administration. The compositions according to the present invention, and in particular compositions comprising progesterone-receptor antagonist (I) or a pharmaceutically acceptable derivative or analogue thereof and pure antiestrogen (I) or pharmaceutically acceptable derivatives or analogues of both compounds, are particularly suitable for oral administration.

It is also envisaged that the mode of administration of the progesterone-receptor antagonist and the pure antiestrogen(s) differ, for example the progesterone-receptor antagonist can be administered subcutaneosly and the pure antiestrogen(s) can be administered orally.

The amounts (a "pharmaceutically effective amount") of the combined active agents to be administered vary within a broad range and depend on the condition to be treated and the mode of administration. They can cover any amount efficient for the intended treatment. Determining a "pharmaceutically effective amount" of the combined active agent is within the purview of a person skilled in the art.

The weight ratio of progesterone-receptor antagonist (I) to the pure antiestrogen(s) (I) as defined above can vary within a broad range. They can either be present in equal amounts or one component can be present in excess of the other component(s). Preferably, 0.1 to 200 mg of the pure antiestrogen (I) and 0.1 to 100 mg of progesterone-receptor antagonist (I) are administered, more preferably 10 to 50 mg of each of the pure antiestrogen (I) and progesterone-receptor antagonist (I). In special cases up to 200 mg of the progesterone-receptor antagonist may be administered. The pure antiestrogen (I) and progesterone-receptor antagonist (I) are preferably present in ratios from 100:1 to 1:100. More preferably, they are present in ratios from 4:1 to 1:4.

The progesterone-receptor antagonist (I) and the pure antiestrogen(s) (I) can be administered either together or separately, at the same time and/or sequentially. Preferably they are administered combined in one unit dose. In case they are administered sequentially, preferably the progesterone-receptor antagonist (I) is administered before the pure antiestrogen(s) (I) as defined above.

The combination of progesterone-receptor antagonist (I) and a pure antiestrogen (I) or pharmaceutically acceptable derivatives or analogues of these components exerts very strong tumor-inhibiting effects in a panel of hormone-dependent breast cancer models (cf. Example 1). In some cases strong inhibition even seems to be synergistic when compared to the inhibition achieved by these compounds alone. The combination of progesterone-receptor antagonist (I) and a pure antiestrogen (I) is furthermore superior to a combination of progesterone-receptor antagonist (I) and the partial antiestrogen tamoxifen, a standard agent in breast cancer therapy (cf. Example 1).

Agents, such as the combination in the various aspects of the invention, that induce apoptosis in cells, for example, in the case of tumor cells, by blocking progression in the G₀G₁-phase, have potential applications for treating and preventing numerous conditions. For example, the combination of progesterone-receptor antagonist (I) and pure antiestrogen(s) (I), may be used for treating those cancers where an indicator of high risk is an increased amount of tumor cells in the S-phase of the cell cycle, such as in breast cancer (see G. M. Clark et al., N. Engl. J. Med. 320, 1989, March, pp.627-633; L. G. Dressler et al., Cancer 61(3), 1988, pp. 420-427 and literature cited therein).

Without limitation to any theory, the results provided in the example indicate that the main mechanism of the antitumor action of the combination of progesterone-receptor antagonist (I) and a pure antiestrogen (I) according to the present invention in the tested model is a direct estrogen-receptor and/or progesterone-receptor-mediated antiproliferative effect at the level of the tumor cells, via the induction of terminal differentiation associated with terminal cell death. In this manner, the combination according to the invention appears to be capable of eliminating the intrinsic block in terminal differentiation inherent in malignant tumor cells in progesterone receptor-positive and estrogen-receptor positive tumors.

The invention is further illustrated in the example. The following example is not to be understood as a limitation.

### Example

### MXT breast cancer model in mice

### Materials and Methods:

MXT mammary tumors obtained from donor mice are implanted in fragments of about 2 mm diameter in the inguinal region of female BDF1 mice (Charles River). Treatment is started when tumors are 25 mm² in size with
A) 1) control, 2) ovariectomy, 3) tamoxifen, 4) pure antiestrogen (Ia), 5) progesterone-receptor antagonist (I), 6) combination of progesterone-receptor antagonist (I) and pure antiestrogen (I), 7) combination of progesterone-receptor antagonist (I) and tamoxifen whereby all compounds are administered 6 times per week subcutaneously or
B) 1) control, 2) ovariectomy, 3) pure antiestrogen (Ia), 4) progesterone-receptor antagonist (I), 5) combination of progesterone-receptor antagonist (I) and pure antiestrogen (I) whereby all compounds are administered 6 times per week orally.

Tumor area is determined by caliper measurements. Tumor weight is determined at the end of the experiment.
Progesterone-receptor antagonist (I) = PA-I: 11β-(4-acetylphenyl)-17β-hydroxy-17α-(1,1,2,2,2-pentafluoroethyl)-estra-4,9-dien-3-one
Pure antiestrogen (Ia) = AE-I: 11β-Fluoro-17α-methyl-7α-{5-[methyl(8,8,9,9,9-pentafluorononyl)amino]pentyl}-estra-1,3,5(10)-triene-3,17β-diol

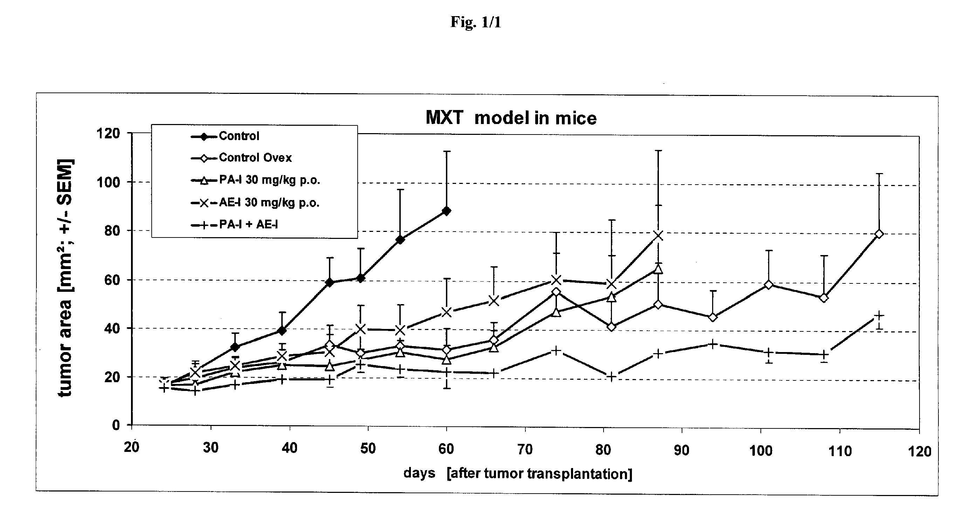

The results for the oral administration of the compounds are shown in Fig. 1. The results for the subcutaneous administration are comparable (data not shown).

Compared to the rapid growth of the control, ovariectomy causes a pronounced tumor growth inhibition. The combination of progesterone-receptor antagonist (I) and pure antiestrogen (Ia) according to the present invention exerts an antitumor effect similar to that of ovariectomy and significantly superior to that of the single compounds. Thus, the tumor growth inhibitory effect of the composition according to the invention can be called a synergistic effect. The composition according to the present invention is superior to tamoxifen, an antiestrogen with partial agonistic properties. This partial agonism of tamoxifen is clearly reflected in its inability to decrease tumor growth in combination. This is even further demonstrated by the weak tumor growth inhibitory effect exerted by a combination of tamoxifen and progesterone-receptor antagonist (I) as compared to the superior effect of the combination according to the present invention, i.e. pure antiestrogen (Ia) (which does not have any agonist properties) and progesterone-receptor antagonist (I).

### Conclusion:

The combination of progesterone-receptor antagonist (I) with a pure antiestrogen (I) according to the present invention proves to be potent in inhibition of the growth of MXT mouse mammary tumors. The combination is superior to the growth inhibition by the single compounds, indicating a synergistic effect.

The combination of progesterone-receptor antagonist (I) with a pure antiestrogen (I) according to the present invention shows also synergistic effects in the treatment of chemically induced tumors (NMU-, DMBA-model) in female rats.
NMU = Nitroso-methyl-urea
DMBA = Dimethyl-benz-anthracene

## Claims

1. Pharmaceutical composition comprising the progesterone-receptor antagonist 11β-(4-acetylphenyl)-17β-hydroxy-17α-(1,1,2,2,2-pentafluoroethyl)-estra-4,9-dien-3-one or a pharmaceutically acceptable derivative or analogue thereof and at least one pure antiestrogen,
wherein the pure antiestrogen is selected from the group of the following compounds:
11β-Fluoro-7α-{5-[methyl(7,7,8,8,9,9,9-heptafluorononyl)amino]pentyl}-17α-methylestra-1,3,5(10)-triene-3,17β-diol N-oxide
11β-Fluoro-7α-{5-[methyl(8,8,9,9,10,10,10-heptafluorodecyl)amino]pentyl}-17α-methylestra-1,3,5(10)-triene-3,17β-diol N-oxide
(RS)-11β-Fluoro-7α-{5-[methyl(7,7,8,8,9,9,10,10,1O-nonafluorodecyl)amino]-pentyl}-17α-methylestra-1,3,5(10)-triene-3,17β-diol N-oxide
11β-Fluoro-7α-{5-[methyl(8,8,9,9,9-pentafluorononyl)amino]pentyl}-17α-methylestra-1,3,5(10)-triene-3,17β-diol N-oxide
11β-Fluoro-7α-{5-[methyl(9,9,10,10,10-pentafluorodecyl)amino]pentyl-17α-methylestra-1,3,5(10)-triene-3,17β-diol N-oxide
11β-Fluoro-7α-{5-[methyl(8,8,9,9,9-pentafluorononyl)amino]pentyl}-17α-methylestra-1,3,5(10)-triene-3,17β-diol
11β-Fluoro-7α-{5-[methyl(7,7,8,8,9,9,10,10,10-nonafluorodecyl)amino]pentyl}-17α-methylestra-1,3,5(10)-trienc-3,17β-diol
11β-Fluoro-7α-{5-[methyl(7,7,8,8,9,9,-heptafluorononyl)amino]pentyl}-17α-methylestra-1,3,5(10)-triene-3,17β-diol
17α-Ethinyl-11β-fluoro-7α-{5-[methyl(7,7,8,8,9,9,10,10,10-nonafluorodecyl)-amino]pentyl}-estra-1,3,5(10)-triene-3,17β-diol
17α-Ethinyl-11β-fluoro-3-(2-tetrahydropyranoyloxy)-7α-{5-[methyl(7,7,8,8,9,9,10,10,10-nonafluorodecyl)amino]pentyl}-estra-1,3,5(10)-trien-17β-ol
11β-Fluoro-3-(2-tetrahydropyranyloxy)-7α-{5-[methyl(7,7,8,8,9,9,10,10,10-nonafluorodecyl)amino]pentyl}-17α-methylestra-1,3,5(10)-trien-17β-ol
11β-Fluoro-7α-{5-[methyl(7,7,8,8,9,9,10,10,10-nonafluorodecyl)amino]pentyl}-17α-trifluoromethylestra-1,3,5(10)-triene-3,17β-diol
11β-Fluoro-7α-{5-[methyl(6,6,7,7,8,8,8-heptafluorooctyl)amino]pentyl}-17α-methylestra-1,3,5(10)-triene-3,17β-diol
11β-Fluoro-7α-{5-[methyl(8,8,9,9,10,10,10-heptafluorodecyl)amino]pentyl}-17α-methylestra- 1,3,5(10)-triene-3,17β-diol
11β-Fluoro-7α-{5-[methyl(6,6,7,7,8,8,9,9,10,10,10-undecafluorodecyl)amino]-pentyl}-17α-methylestra-1,3,5(10)-triene-3,17β-diol
11β-Fluoro-7α-{5-[methyl(5,5,6,6,7,7,8,8,8-nonafluorooctyl)amino]pentyl}-17α-methylestra-1,3,5(10)-triene-3,17β-diol
11β-Fluoro-7α-{5-[methyl(9,9,10,10,11,11,11-heptafluoroundecyl)amino]-pentyl}-17α-methylestra-1,3,5(10)-triene-3,17β-diol
11β-Fluoro-7α-{5-[methyl(9,9,10,10,10-pentafluorodecyl)amino]pentyl}-17α-methylestra-1,3,5(10)-triene-3,17β-diol;
whereby pharmacologically compatible acid addition salts as well as esters are also included.

2. The pharmaceutical composition according to claim 1, wherein the pure antiestrogen is 11β-Fluoro-17α-methyl-7α-{5-[methyl(8,8,9,9,9-pentafluorononyl)amino]pentyl}-estra-1,3,5(10)-triene-3,17β-diol or a pharmaceutically acceptable derivative or analogue thereof.

3. The pharmaceutical composition according to any one of the preceding claims wherein the weight ratio of the progesterone-receptor antagonist and the pure antiestrogen is from 1:100 to 100:1.

4. The pharmaceutical composition according to any one of the preceding claims wherein the weight ratio of the progesterone-receptor antagonist and the pure antiestrogen is from 1:4 to 4:1.

5. The pharmaceutical composition according to any one of the preceding claims wherein the progesterone-receptor antagonist is present in a unit dose of 0.1 to 100 mg and the pure antiestrogen is present in a unit dose of 0.1 to 200 mg.

6. The pharmaceutical composition according to any one of the preceding claims wherein the progesterone-receptor antagonist is present in a unit dose of 10 to 50 mg and the pure antiestrogen is present in a unit dose of 10 to 50 mg.

7. Pharmaceutical Composition according to any of the proceeding claims as a medicament for the prophylaxis or treatment of a hormone-dependent disease in a mammal.

8. Pharmaceutical Composition according to claim 7 as a medicament for treatment of breast cancer.
